# FASCICULE DE BREVET EUROPEEN

(11) **EP 1 066 055 B1**
(45) Date de publication et mention de la délivrance du brevet: **14.02.2007**
(21) Numéro de dépôt: 99909069.9
(22) Date de dépôt: 22.03.1999
(51) Int. Cl.: A61K 39/39, A61K 48/00

(54) **Vaccins à ADN adjuvés avec des polymères d'acide acrylique ou méthacrylique ou d' EMA (R) pour les chevaux**
DNS Pferdeimpfstoffe adjuviert mit Acryl- oder Methacrylpolymersäuren oder EMA (R)
Horse DNA vaccines containing the adjuvant acrylic acid, methacrylic acid or EMA (R)

(30) Priorité: 03.04.1998 FR 9804409
(43) Date de publication de la demande: 10.01.2001
(73) Titulaire: MERIAL, 69002 Lyon (FR)
(72) Inventeur: AUDONNET, Jean-Christophe, Francis, F-69006 Lyon (FR); MINKE, Jules, Maarten, F-69960 Corbas (FR)
(74) Mandataire: Nargolwalla, Cyra
(86) Numéro de dépôt international: PCT/FR1999/000666
(87) Numéro de publication internationale: WO 1999/051269

(56) Documents cités:
- EP-A- 0 283 085
- WO-A-95/11700
- WO-A-96/20007

## Description

La présente invention concerne un perfectionnement aux vaccins ADN, encore appelés vaccins plasmidiques ou polynucléotidiques, comprenant et exprimant in vivo un ou plusieurs gènes hétérologues. Elle a trait en particulier à de tels vaccins perfectionnés, à l'utilisation de composés adjuvants particuliers pour la mise en oeuvre de tels vaccins ainsi qu'aux méthodes de vaccination y relatives. Elle a encore pour objet un procédé de préparation de ces vaccins.

Les demandes de brevet WO-A-90 1 1092, WO-A-93 19183, WO-A-94 21797, WO-A-95 11307 et WO-A-95 20660 ont fait usage de la technique récemment développée des vaccins polynucléotidiques. On sait que ces vaccins utilisent un plasmide apte à exprimer dans les cellules de l'hôte un gène inséré dans le plasmide et codant pour un immunogène. Toutes les voies d'administration ont été proposées (intrapéritonéale, intraveineuse, intramusculaire, transcutanée, intradermique, mucosale, etc.). Différents moyens de vaccination peuvent également être utilisés, tels que ADN déposé à la surface de particules d'or et projeté de façon à pénétrer dans les cellules de la peau de l'animal (Tang et al,. Nature 356, 152-154, 1992) et les injecteurs par jet liquide permettant de transfecter à la fois dans la peau, le muscle, les tissus graisseux et les tissus mammaires (Furth et al., Analytical Biochemistry, 205, 365-368, 1992).

On peut utiliser ces vaccins polynucléotidiques sous forme d'ADN nu ou sous forme de complexe avec des liposomes ou des lipides cationiques.

L'invention a pour objectif d'améliorer l'efficacité des vaccins ADN en proposant de nouvelles formulations vaccinales simples et faciles à préparer.

Elle a aussi pour objectif de fournir une telle solution n'entraînant pas d'interactions fortes entre l'ADN et le tiers ingrédient, susceptibles de conduire à la formation d'un complexe.

Elle a aussi pour objectif de proposer une telle solution permettant soit par simple mélange de préparer des vaccins stables, formulés sous une forme liquide, soit de réaliser de manière aisée un vaccin liquide par mélange extemporané.

La déposante a trouvé de manière surprenante que les composés de la classe des carbomères répondent à ces divers objectifs et notamment sont en mesure d'adjuver de manière simple mais dans des proportions très avantageuses les vaccins ADN nus.

La présente invention a donc pour objet un vaccin ADN comprenant un ADN nu, en particulier plasmide vaccinal circulaire, super-enroulé ou non, ou une molécule d'ADN linéaire, incorporant et exprimant in vivo une séquence nucléotidique codant pour un polypeptide antigénique, de préférence un gène d'un agent pathogène, et au moins un composé adjuvant choisi parmi les polymères de l'acide acrylique ou méthacrylique et les copolymères d'anhydride maléique et de dérivé alcényle.

Par ADN nu, on entend comme cela est aujourd'hui communément accepté, une unité de transcription ADN sous forme d'une séquence polynucléotidique comprenant au moins une séquence nucléotidique codant pour un polypeptide antigénique ou antigène d'une valence et les éléments nécessaires à son expression in vivo. On préfère la forme plasmide circulaire, super-enroulée ou non. Par valence, dans la présente invention, on entend au moins un antigène assurant une protection contre un pathogène, la valence pouvant contenir, à titre de sous-valence, un ou plusieurs gènes naturels ou modifiés, d'une ou plusieurs souches du pathogène considéré.

Les composés adjuvants préférés sont les polymères de l'acide acrylique ou méthacrylique réticulés, notamment par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 (incorporé par référence) décrivant de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituants, tel que méthyl. Les produits vendus sous la dénomination Carbopol® (BF Goodrich, Ohio, USA) sont particulièrement appropriés. Ils sont réticulés par un allyl saccharose ou par de l'allylpentaérythritol. Parmi eux, on peut citer les Carbopol® 974P, 934P et 971 P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186 : 778-780, 4 juin 1960 (incorporé par référence).

Sur le plan de leur structure, les polymères d'acide acrylique ou méthacrylique et les EMA® sont formés de préférence de motifs de base de formule suivante : 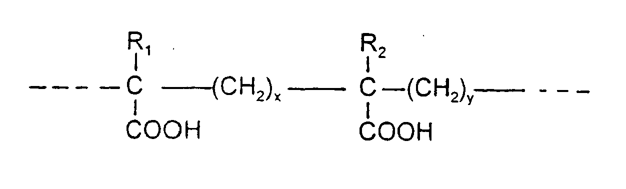 dans laquelle :
- R₁ et R₂, identiques ou différents, représentent H ou CH₃
- x = 0 ou 1, de préférence x = 1
- y = 1 ou 2, avec x+y = 2

Pour les EMA®, x = 0 et y = 2. Pour les carbomères, x = y = 1.

La dissolution de ces polymères dans l'eau conduit à une solution acide qui sera neutralisée, de préférence jusqu'à pH physiologique, pour donner la solution adjuvante dans laquelle le vaccin proprement dit sera incorporé. Les groupes carboxyliques du polymère sont alors en partie sous forme COO⁻.

De manière préférée, on réalise une solution d'adjuvant selon l'invention, notamment de carbomère, dans de l'eau distillée, de préférence en présence de chlorure de sodium, la solution obtenue étant à pH acide. On dilue cette solution mère en l'ajoutant dans la quantité nécessaire (pour l'obtention de la concentration finale souhaitée), ou une partie importante de celle-ci, d'eau chargée en NaCl, de préférence eau physiologique (NaCl 9 g/l), en une ou plusieurs fois avec neutralisation concomitante ou subséquente (pH 7.3 à 7,4), de préférence par NaOH. Cette solution à pH physiologique sera utilisée telle quelle pour mélange avec le vaccin, notamment conservé sous forme lyophilisée, liquide ou congelée.

La concentration en polymère dans la composition vaccinale finale sera de 0,01 % à 2 % P/V, plus particulièrement de 0,06 à 1 % P/V, de préférence de 0,1 à 0,6 % P/V.

Pour la vaccination des porcs, l'invention peut s'appliquer notamment à la vaccination contre le virus de la maladie d'Aujeszky (virus PRV ou pseudorage), virus de la grippe porcine (virus Influenza porcin, SIV), virus de la maladie mystérieuse du porc (virus PRRS), virus de la parvovirose porcine (virus PPV), virus de la peste porcine classique (virus HCV, ou Hog Cholera Virus) et bactérie responsable de l'actinobacillose (A. pleuropneumoniae). Les plasmides utilisables dans l'invention comprennent, pour chaque valence, un ou plusieurs des gènes codant pour des immunogènes majeurs des agents pathogènes considérés. On peut citer en particulier les gènes gB et gD pour le virus de la maladie d'Aujeszky, les gènes HA, NP, N pour le virus de la grippe porcine, les gènes ORF5 (E), ORF3, ORF6 (M) pour le virus PRRS, VP2 pour le virus de la parvovirose, E2, E1 + E2, E1 + E2 + C pour le virus de la peste porcine classique et apxl, apxll et apxlll pour A. pleuropneumoniae. De manière particulièrement avantageuse, on pourra se reporter aux formules de vaccins polynucléotidiques décrites dans la demande de brevet incorporée par référence WO-A-98 03 658 (FR-A-2 751 224) qui concerne des vaccins contre les pathologies respiratoires et de reproduction des porcs. Cette demande décrit notamment un certain nombre de plasmides qui pourront être directement utilisés à titre d'exemples dans le cadre de la présente invention en combinaison avec un adjuvant selon l'invention. L'homme du métier pourra ainsi combiner, aux adjuvants conformes à l'invention, les plasmides spécifiquement décrits dans cette demande antérieure, à savoir pAB090 comprenant le gène gB du virus PRV, pPB098 comprenant le gène gD du virus PRV, pPB143 comprenant le gène HA de la grippe porcine, souche H1N1, pPB142 comprenant le gène NP grippe porcine de souche H1N1, pPB144 comprenant le gène HA de la grippe porcine, souche H3N2, pPB132 comprenant le gène NP de la grippe porcine, souche H3N2, pAB025 comprenant l'ORF5 du virus PRRS, souche Lelystad, pAB001 comprenant l'ORF5 du virus PRRS, souche USA, pAB091 comprenant l'ORF3 du virus PRRS, souche Lelystad, pAB092 comprenant l'ORF3 du virus PRRS, souche USA, pAB004 comprenant le gène VP2 du parvovirus porcin, pAB069 comprenant le gène E1 du virus de la peste porcine (HCV), pAB061 comprenant le gène E2 du virus de la peste porcine (HCV), pAB162 comprenant le gène apxl délété d'A. pleuropneumoniae, pPB163 comprenant le gène apxll délété d'A. pleuropneumoniae, pPB174', pPB189 et pPB190 comprenant le gène apxIII délété d'A. pleuropneumoniae.

Pour la vaccination des chevaux, on peut citer en particulier la vaccination contre le virus de la rhinopneumonie équine (EHV), notamment de type 1 (EHV-1) et de type 4 (EHV-4), contre le virus de la grippe équine EIV, contre le tétanos (Cl. tetani) contre le virus de l'encéphalite de l'Est (EEV), de l'encéphalite de l'Ouest (WEV) et de l'encéphalite vénézuélienne (VEV), ou encore contre la maladie de la Lyme (B. burgdorferi), contre l'artérite équine (EAV) et contre la rage. Parmi les gènes codant pour des immunogènes majeurs utilisables selon l'invention, on peut citer des gènes gB et gD pour la valence rhinopneumonie équine, notamment de types 1 et 4, les gènes HA, NA et NP pour la grippe équine, la sous-unité C, éventuellement modifiée par mutation ou délétion, pour la valence tétanos, les gènes C et E2 pour les encéphalites, les gènes OspA, OspB et p100 pour la maladie de Lyme, les gènes E, M et N pour l'artérite équine, le gène G pour la rage. De telles formules de vaccins polynucléotidiques contre les pathologies du cheval sont décrites notamment dans la demande de brevet incorporée par référence WO-A-98 03 198 (FR-A-2 751 226). Cette même demande décrit un certain nombre de plasmides directement utilisable dans la présente invention en combinaison avec un adjuvant conforme à l'invention. L'homme de l'art pourra donc combiner, à l'adjuvant conforme à l'invention, un plasmide tel que décrit précisément dans cette demande, à savoir pAB042 comprenant le gène gB du virus EHV-1, pAB031 comprenant le gène gB du virus EHV-4, pAB013 comprenant le gène gD du virus EHV-1, pAB032 comprenant le gène gD du virus EHV-4, pAB043 comprenant le gène HA de la grippe équine, souche Prague, pAB033 comprenant le gène HA de la grippe équine, souche Suffolk, pAB099 comprenant le gène HA de la grippe équine, souche Fontainebleau, pAB085 comprenant le gène NP de la grippe équine, souche Prague, pAB084 comprenant le gène NP de la grippe équine, souche Jillin, pAB070 comprenant le gène de la sous-unité C de la toxine tétanique, pAB017 comprenant le gène OspA de Borrelia burgdorferi, pAB094 comprenant le gène E2 du virus de l'encéphalite de l'Est, pAB093 comprenant le gène C du virus de l'encéphalite de l'Est, pAB096 comprenant le gène E2 de l'encéphalite de l'Ouest, pAB095 comprenant le gène C du virus de l'encéphalite de l'Ouest, pAB098 comprenant le gène E2 du virus de l'encéphalite vénézuélienne, pAB097 comprenant le gène C du virus de l'encéphalite vénézuélienne, pAB041 comprenant le gène G du virus de la rage.

Pour la vaccination des chiens, l'invention peut s'appliquer notamment à la vaccination contre le virus de la maladie de Carré (CDV), le parvovirus canin (CPV), le coronavirus canin (CCV), le virus herpès canin (CHV), la maladie de Lyme, la rage. Parmi les gènes codant pour des immunogènes majeurs utilisables dans le cadre de la présente invention, on peut citer tout particulièrement les gènes HA, F, M et N pour le virus de la maladie de Carré, le gène VP2 pour le parvovirus canin, les gènes S et M pour le coronavirus canin (CCV), les gènes gB et gD pour le virus CHV, les gènes OspA et OspB et p100 pour B. burgdorferi (maladie de Lyme), le gène G pour la rage. De telles formules de vaccins polynucléotidiques sont décrites en particulier dans la demande de brevet incorporée par référence WO-A-98 03 199 (FR-A-2 751 227). L'homme du métier pourra donc se reporter aux plasmides décrits dans cette demande, en combinaison avec les adjuvants conformes à l'invention. Tout particulièrement, il pourra combiner, aux adjuvants conformes à l'invention, les plasmides spécifiques décrits dans cette demande, à savoir pAB044 comprenant le gène HA de CDV, pAB036 comprenant le gène F de CDV, pAB024 comprenant le gène VP2 du parvovirus canin, pAB021 comprenant le gène S de CCV, pAB022 comprenant le gène M de CCV, pAB037 comprenant le gène gB de CHV, pAB038 comprenant le gène gD de CHV, pAB017 comprenant le gène OspA de B. burgdorferi, pAB041 comprenant le gène G du virus de la rage.

Pour la vaccination des bovins, l'invention peut s'appliquer notamment à la vaccination contre le virus herpès bovin de type 1 ou 5 (BHV-1 et BHV-5, responsable de la forme nerveuse de la maladie), le virus respiratoire syncytial bovin (BRSV), le virus de la maladie des muqueuses ou pestivirus bovin (BVD), le virus parainfluenza bovin de type 3 (BPI-3). Parmi les gènes codant pour les immunogènes majeurs permettant la vaccination contre ces virus, on peut citer en particulier les gènes gB et gD pour le virus herpès bovin, F et G pour le virus respiratoire syncytial bovin, E2, C + E1 + E2 et E1 + E2 pour le virus de la maladie des muqueuses, HN et F pour le virus parainfluenza bovin de type 3. De telles formules de vaccins sont décrites en particulier dans la demande de brevet incorporée par référence WO-A-98 03 200 (FR-A-2 751 229). L'homme du métier pourra donc utiliser les plasmides décrits dans cette demande en combinaison avec les adjuvants conformes à l'invention. En particulier il pourra combiner aux adjuvants conformes à l'invention les plasmides spécifiquement décrits dans cette demande, à savoir pPB156 comprenant le gène gB de BHV-1, pAB087 comprenant le gène gD de BHV-1, pAB011 comprenant le gène F de BRSV, pAB012 comprenant le gène G de BRSV, pAB058 comprenant le gène C de BVD, pAB059 comprenant le gène E1 de BVD, pAB060 comprenant le gène E2 de BVD, pAB071 comprenant le gène HN de BPI-3, pAB072 comprenant le gène F de BPI-3.

Pour la vaccination des chats, l'invention peut s'appliquer notamment à la vaccination contre le virus de leucémie féline FeLV, notamment sous-types A et B, le virus de la panleucopénie féline (FPV), le virus de la péritonite infectieuse féline (FIPV), le virus du coryza ou herpèsvirus félin (FHV), le virus de la calicivirose féline (FCV), le virus de l'immunodéficience féline (FIV) et le virus de la rage (rhabdovirus). Parmi les gènes codant pour des immunogènes majeurs permettant la vaccination contre ces pathogènes, on peut citer en particulier les gènes env et gag/pol pour la leucémie féline, VP2 pour la panleucopénie, S modifié (FR-A-2 724 385 incorporé par référence) et M pour la péritonite infectieuse, gB et gD pour le coryza, capside pour la calicivirose, env et gag/pro pour l'immunodéficience féline et G pour la rage. Des formules de vaccins polynucléotidiques sont ainsi décrites dans la demande de brevet incorporée par référence WO-A-98 03 660 (FR-A-2 751 223). L'homme du métier pourra combiner des plasmides tels que décrits dans cette demande aux adjuvants conformes à l'invention. En particulier, il pourra combiner, aux adjuvants conformes à l'invention, les plasmides spécifiquement décrits dans cette demande, à savoir pPB179 comprenant le gène env du virus FeL V-A, pPB180 comprenant le gène env du virus FeLV-B, pPB181 comprenant le gène gag/pol de FeL V-A, pAB009 comprenant le gène VP2 de FPV, pAB053 comprenant le gène S modifié du virus FIPV, pAB052 comprenant le gène M de FIPV, pAB056 comprenant le gène N de FIPV, pAB028 comprenant le gène gB de FHV, pAB029 comprenant le gène gD de FHV, pAB010 comprenant le gène C de FCV, pAB030 comprenant le gène env de FIV, pAB083 comprenant le gène gag/pro de FIV, pAB041 comprenant le gène G du virus de la rage.

Pour la vaccination des espèces aviaires, l'invention peut s'appliquer notamment à la vaccination contre le virus de la maladie de Marek (MDV), le virus de la maladie de Newcastle (NDV), le virus de la maladie de Gumboro (IBDV ou Infectious Bursal Disease Virus), le virus de la bronchite infectieuse (IBV ou virus de la bronchite infectieuse), le virus de l'anémie infectieuse (CAV), le virus de la laryngotrachéite infectieuse (ILTV), le virus de l'encéphalomyélite (AEV ou encore virus de la leucose aviaire ALV), le virus de la pneumovirose ou pneumovirus, et le virus de la peste aviaire ou grippe aviaire. Parmi les gènes codant pour les immunogènes majeurs utilisables dans la présente invention, on peut citer tout particulièrement les gènes gB et gD pour le virus de la maladie de Marek, HN et F pour le virus de la maladie de Newcastle, VP2 pour le virus de la maladie de Gumboro, S, M et N pour le virus de la bronchite infectieuse, C + NS1 pour le virus de l'anémie infectieuse, gB et gD pour le virus de la laryngotrachéite infectieuse, env et gag/pro pour le virus de l'encéphalomyélite, F et G pour le virus de la pneumovirose et HA, N et NP pour le virus de la peste aviaire. De telles formules de vaccins polynucléotidiques sont décrites dans la demande de brevet incorporée par référence WO-A-98 03 659 (FR-A-2 751 225). L'homme du métier pourra donc se reporter aux plasmides décrits dans cette demande en vue de les combiner aux adjuvants conformes à l'invention. Tout particulièrement l'homme du métier pourra combiner aux adjuvants conformes à l'invention les plasmides décrits spécifiquement dans cette demande, à savoir pAB045 comprenant le gène gB de MDV, pAB080 comprenant le gène gD de MDV, pAB046 comprenant le gène HN de NDV, pAB047 comprenant le gène F de NDV, pAB048 comprenant le gène VP2 d'IBDV, pAB049 comprenant le gène S1 d'IBV, pAB050 comprenant le gène M d'lBV, pAB051 comprenant le gène N d'lBV. pAB054 comprenant le gène VP1 de CAV, pAB055 comprenant le gène VP2 de CAV, pAB076 comprenant le gène gB d'ILTV, pAB089 comprenant le gène gD d'ILTV, pAB086 comprenant le gène env d'AEV, pAB081 comprenant le gène gag/pro d'AEV, pAB082 comprenant le gène G du pneumovirus, pAB077 comprenant le gène HA de la peste aviaire, souche H2N2, pAB078 comprenant le gène HA de la peste aviaire, souche H7N7, pAB088 comprenant le gène NP de la peste aviaire, souche H1N1, pAB079 comprenant le gène N de la peste aviaire, souche H7N1.

Chaque ADN nu en particulier plasmide, comprend un promoteur apte à assurer, dans les cellules hôtes, l'expression du gène inséré sous sa dépendance. Il s'agira en général d'un promoteur eucaryote fort et en particulier d'un promoteur précoce du cylomégalovirus CMV-IE, d'origine humaine ou murine, ou encore éventuellement d'une autre origine telle que rat, cochon, cobaye. De manière plus générale, le promoteur pourra être soit d'origine virale, soit d'origine cellulaire. Comme promoteur viral autre que CMV-lE, on peut citer le promoteur précoce ou tardif du virus SV 40 ou le promoteur LTR du virus du Sarcome de Rous. Il peut aussi s'agir d'un promoteur du virus dont provient le gène, par exemple le promoteur propre du gène. Comme promoteur cellulaire, on peut citer le promoteur d'un gène du cytosquelette, tel que par exemple le promoteur de la desmine (Bolmont et al., Journal of Submicroscopic Cytology and Pathology, 1990, 22, 117-122 ; et ZHENLIN et al., Gene, 1989, 78, 243-254), ou encore le promoteur de l'actine. Lorsque plusieurs gènes sont présents dans le même ADN nu, en particulier plasmide, ceux-ci peuvent être présentés dans la même unité de transcription ou dans deux unités différentes.

Bien entendu, un vaccin pourra combiner, pour chacune des valences décrites ci-dessus, plusieurs gènes au sein d'un même ADN nu, en particulier plasmide, et/ou plusieurs ADN nus, en particulier plasmides, comprenant chacun un ou plusieurs gènes du même virus.

L'invention a aussi pour objet des vaccins recombinants multi-valents, c'est-à-dire contenant un, ou de préférence deux ou plusieurs, ADN nus, en particulier plasmides, exprimant des antigènes de deux ou plusieurs maladies, en mélange dans une solution adjuvante conforme à l'invention.

Dans le vaccin prêt-à-l'emploi, l'ADN nu, en particulier le plasmide vaccinal, est présent dans les quantités habituellement utilisées et décrites dans la littérature.

L'invention a aussi pour objet une méthode de vaccination consistant à administrer par voie parentérale, de préférence intramusculaire, intradermique, ou par voie mucosale, un vaccin ADN conforme à l'invention, à raison d'une ou plusieurs administrations.

L'invention a encore pour objet l'utilisation des composés adjuvants conformes à l'invention pour la réalisation de vaccins ADN adjuvés tels que décrits ici.

L'invention va être maintenant décrite plus en détails, à l'aide de modes de réalisation pris à titre d'exemples non limitatifs, et se référant aux figures annexées.

### Liste des Figures :

| | |
|---|---|
| Figure N°1 : | Séquence du gène hémagglutinine (HA) du virus de la grippe équine souche Newmarket 2/93 |
| Figure N°2 : | Séquence du gène hémagglutinine (HA) du virus de la grippe équine souche Kentucky 1/94 |
| Figure N°3 : | Séquence du gène neuraminidase (NA) du virus de la grippe équine souche Newmarket 2/93 |
| Figure N°4 : | Séquence du gène neuraminidase (NA) du virus de la grippe équine souche Kentucky 1/94 |
| Figure N°5 : | Séquence du gène nucléoprotéine (NP) du virus de la grippe équine souche Newmarket 2/93 |
| Figure N°6 : | Séquence du gène nucléoprotéine (NP) du virus de la grippe équine souche Kentucky 1/94 |

### Liste des Séquences :

| | |
|---|---|
| SEO ID N°1 : | Oligonucléotide CCL007 |
| SEQ ID N°2 : | Oligonucléotide CCL018 |
| SEQ ID N°3 : | Séquence du gène HA souche EIV Newmarket 2/93 |
| SEO ID N°4 : | Oligonucléotide CCL020 |
| SEQ ID N°5 : | Séquence du gène HA souche EIV Kentucky 1/94 |
| SEQ ID N°6 : | Oligonucléotide AB260 |
| SEQ ID N°7 : | Oligonucléotide AB262 |
| SEQ ID N°8 : | Séquence du gène NA souche EIV Newmarket 2/93 |
| SEO ID N°9 : | Séquence du gène NA souche EIV Kentucky 1/94 |
| SEQ ID N°10 : | Oligonucléotide CCL019 |
| SEQ ID N°11 : | Oligonucléotide CCL021 |
| SEQ ID N°12 : | Séquence du gène NP souche EIV Newmarket 2/93 |
| SEO ID N°13 : | Séquence du gène NP souche EIV Kentucky 1/94 |

### Exemple 1 : Adjuvant

Le carbomère utilisé dans les vaccins conformes à la présente invention est le Carbopol® 974P fabriqué par la société BF Goodrich (PM environ 3 millions).

On prépare tout d'abord une solution mère à 1,5 % de Carbopol® 974P dans de l'eau distillée contenant du chlorure de sodium à 1 g/l.

Cette solution mère est ensuite utilisée pour la fabrication d'une solution de Carbopol® en eau physiologique à 4 mg/ml. La solution mère est versée dans la totalité de l'eau physiologique (ou éventuellement dans la majorité de celle-ci) en 1 fois ou éventuellement en plusieurs fois avec à chaque fois l'ajustement du pH à l'aide de NaOH (par exemple 1 N ou plus concentré) à une valeur d'environ 7,3 à 7,4.

On obtient alors une solution de Carbopol® prête à l'emploi.

### Exemple 2 : Culture des virus

Les virus sont cultivés sur le système cellulaire approprié jusqu'à obtention d'un effet cytopathique. Les systèmes cellulaires à utiliser pour chaque virus sont bien connus de l'homme du métier. Brièvement, des cellules sensibles au virus utilisé, cultivées en milieu minimum essentiel de Eagle (milieu "MEM) ou un autre milieu approprié, sont inoculées avec la souche virale étudiée en utilisant une multiplicité d'infection de 1. Les cellules infectées sont alors incubées à 37°C pendant le temps nécessaire à l'apparition d'un effet cytopathique complet (en moyenne 36 heures).

### Exemple 3 : Extraction des ADNs génomiques viraux

Après culture, le surnageant et les cellules lysées sont récoltées et la totalité de la suspension virale est centrifugée à 1000 g pendant 10 minutes à + 4°C pour éliminer les débris cellulaires. Les particules virales sont alors récoltées par ultracentrifugation à 400000 g pendant 1 heure à + 4°C. Le culot est repris dans un volume minimum de tampon (Tris 10 mM, EDTA 1 mM). Cette suspension virale concentrée est traitée par la protéinase K (100 µg/ml final) en présence de sodium dodecyl sulfate (SDS) (0,5% final) pendant 2 heures à 37°C. L'ADN viral est ensuite extrait avec un mélange de phénol/chloroforme, puis précipité avec 2 volumes d'éthanol absolu. Après une nuit à - 20°C, l'ADN est centrifugé à 10000 g pendant 15 minutes à + 4°C. Le culot d'ADN est séché, puis repris dans un volume minimum d'eau ultrapure stérile. Il peut alors être digéré par des enzymes de restriction.

### Exemple 4 : Isolement des ARNs génomiques viraux

Les virus à ARN ont été purifiés selon les techniques bien connues de l'homme du métier. L'ARN viral génomique de chaque virus a été ensuite isolé en utilisant la technique d'extraction "thiocyanate de guanidium/phénol-chloroforme" décrite par P. Chomczynski et N. Sacchi (Anal. Biochem. 1987. **162.** 156-159).

### Exemple 5 : Techniques de biologie moléculaire

Toutes les constructions de plasmides ont été réalisées en utilisant les techniques standards de biologie moléculaire décrites par J. Sambrook *et al. (Molecular Cloning: A Laboratory Manual.* 2^{nd} Edition. Cold Spring Harbor Laboratory. Cold Spring Harbor. New York. 1989). Tous les fragments de restriction utilisés pour la présente invention ont été isolés en utilisant le kit "Geneclean" (BIO101 Inc. La Jolla, CA).

### Exemple 6 : Technique de RT-PCR

Des oligonucléotides spécifiques (comportant à leurs extrémités 5' des sites de restriction pour faciliter le clonage des fragments amplifiés) ont été synthétisés de telle façon qu'ils couvrent entièrement les régions codantes des gènes devant être amplifiés (voir exemples spécifiques). La réaction de transcription inverse (RT) et l'amplification en chaîne par polymérase (PCR) ont été effectuées selon les techniques standards (J. Sambrook *et al. Molecular Cloning: A Laboratory Manual.* 2^{nd} Edition. Cold Spring Harbor Laboratory. Cold Spring Harbor. New York. 1989). Chaque réaction de RT-PCR a été faite avec un couple d'amplimers spécifiques et en prenant comme matrice l'ARN génomique viral extrait. L'ADN complémentaire amplifié a été extrait au phénol/chloroforme/alcool isoamylique (25:24:1) avant d'être digéré par les enzymes de restriction.

### Exemple 7 : plasmide pVR1012

Le plasmide pVR1012 a été obtenu auprès de Vical Inc. San Diego, CA, USA. Sa construction a été décrite dans J. Hartikka *et al.* (Human Gene Therapy. 1996. **7.** 1205-1217, incorporé par référence).

### Exemple 8 : Construction du plasmide pCCL027 (gène EIV HA Newmarket 2/93)

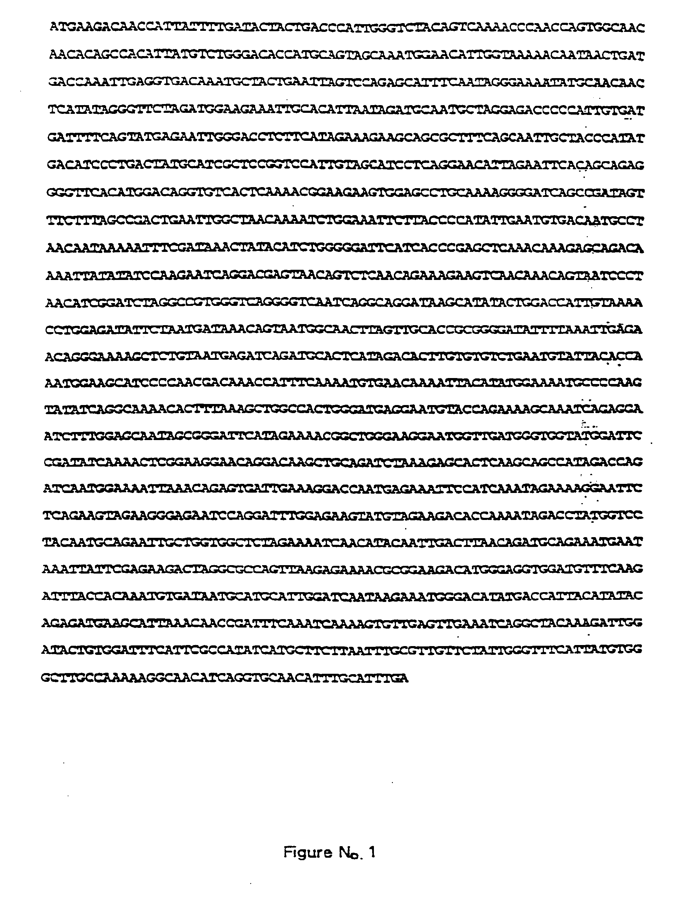

Une réaction de RT-PCR selon la technique décrite dans l'exemple 6 a été réalisée avec l'ARN génomique du virus de la grippe équine (Equine Influenza Virus ou EIV)(souche Newmarket 2/93)(Daly *et al.* J. Gen. Virol. 1996. 77. 661-671), préparé selon la technique décrite dans l'exemple 4, et avec les oligonucléotides suivants :
CCL007 (40 mer)(SEQ ID N°1)
   5' TTGTCGACTCAATCATGAAGACAACCATTATTTTGATACT 3'
CCL018 (34 mer)(SEQ ID N°2)
   5' TTGGATCCTTACTCAAATGCAAATGTTGCACCTG 3'
pour isoler le gène codant pour la glycoprotéine HA du virus de la grippe équine (souche Newmarket 2/93) (Figure N°1, SEQ ID N°3) sous la forme d'un fragment PCR d'environ 1750 pb. Ce fragment a été purifié, puis ligaturé avec le vecteur pCRII (Cat# K2000-01. In Vitrogen Corp. Carlsbad, CA) pour donner le plasmide pCCL026. Le plasmide pCCL026 a alors été digéré avec les enzymes de restriction SalI et NotI pour isoler un fragment Sall-Notl de 1751 pb contenant le gène EIV HA Newmarket 2/93. Ce fragment a ensuite été ligaturé avec le plasmide pVR1012 (voir exemple 7), préalablement digéré par SalI et Notl, pour donner le plasmide pCCL027 (6642 pb).

### Exemple 9 : Construction du plasmide pPB242 (gène EIV HA Kentucky 1/94)

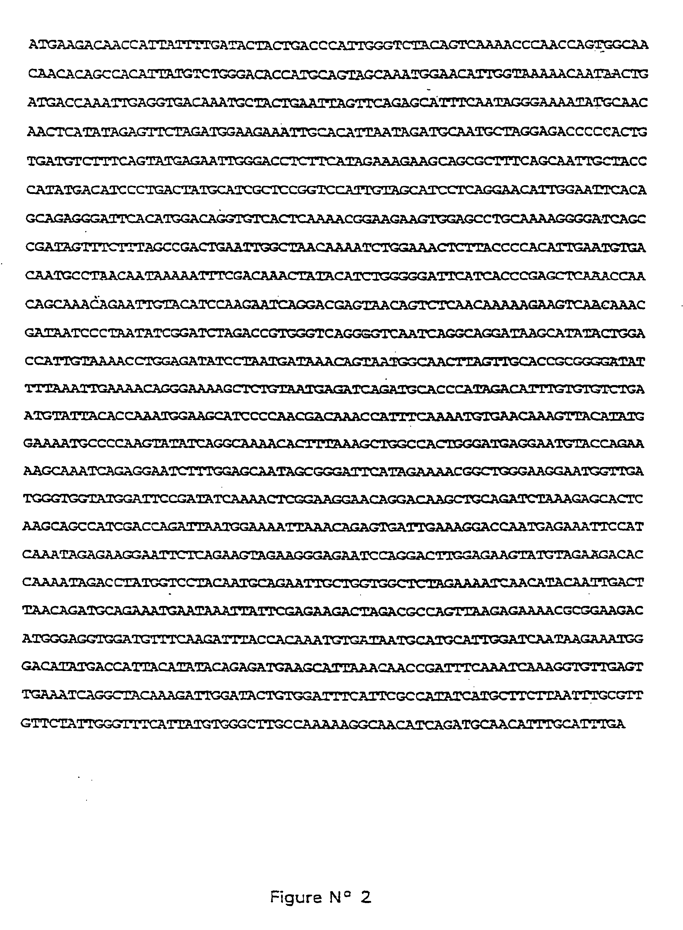

Une réaction de RT-PCR selon la technique décrite dans l'exemple 6 a été réalisée avec l'ARN génomique du virus de la grippe équine (Equine Influenza Virus ou EIV)(souche Kentucky 1/94)(Daly *et al.* J. Gen. Virol. 1996. **77.** 661-671, préparé selon la technique décrite dans l'exemple 4, et avec les oligonucléotides suivants:
CCL007 (40 mer) (SEQ ID N°1)
   5' TTGTCGACTCAATCATGAAGACAACCATTATTTTGATACT 3'
CCL020 (34 mer)(SEQ ID N°4)
   5' TTGGATCCTTACTCAAATGCAAATGTTGCATCTG 3'
pour isoler le gène codant pour la glycoprotéine HA du virus de la grippe équine (souche Kentucky 1/94) (Figure N°2, SEQ ID N°5) sous la forme d'un fragment PCR d'environ 1750 pb. Ce fragment a été purifié, puis ligaturé avec le vecteur pCRll (Cat# K2000-01, InVitrogen Corp Carlsbad, CA) pour donner le plasmide pCCL028. Le plasmide pCCL028 a été digéré avec les enzymes de restriction SacI et BamHl pour isoler un fragment Sacl-BamHl de 1153 pb (fragment A) contenant la partie 3' du gène EIV HA Kentucky 1/94. Le plasmide pCCL028 a été digéré avec les enzymes de restriction Sacl et EcoRV pour isoler un fragment Sacl-EcoRV de 621 pb (fragment B) contenant la partie 5' du gène EIV HA Kentucky 1/94. Les fragments A et B ont ensuite été ligaturés ensemble avec le plasmide pVR1012 (voir exemple 7), préalablement digéré par EcoRV et BamHl, pour donner le plasmide pPB242 (6688 pb).

### Exemple 10 : Construction du plasmide pAB142 (gène EIV NA Newmarket 2/93)

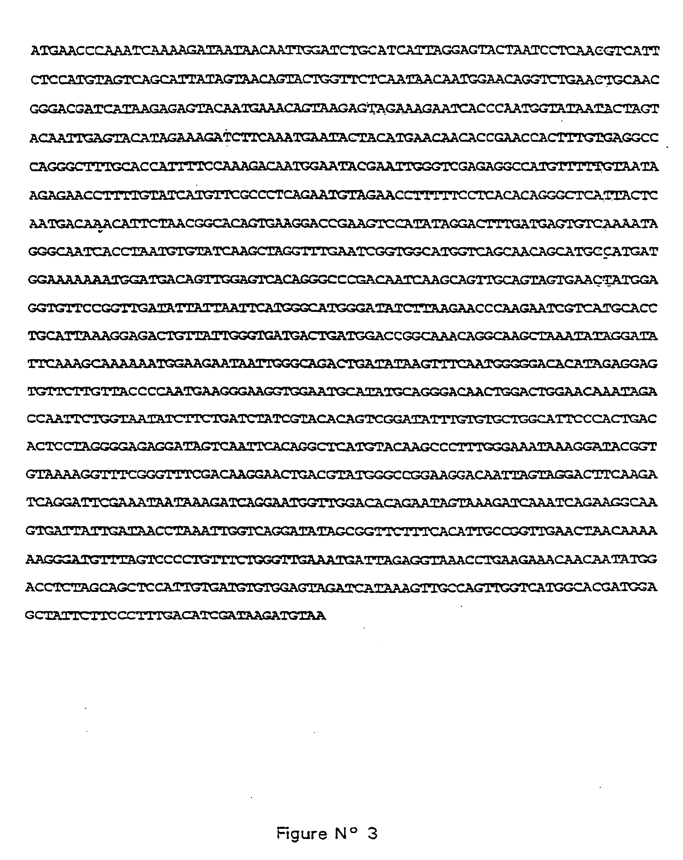

Une réaction de RT-PCR selon la technique décrite dans l'exemple 6 a été réalisée avec l'ARN génomique du virus de la grippe équine (Equine Influenza Virus ou EIV)(souche Newmarket 2/93)(Daly *et al.* J. Gen. Virol. 1996. **77.** 661-671, préparé selon la technique décrite dans l'exemple 4, et avec les oligonucléotides suivants :
AB260 (35 mer) (SEQ ID N°6)
   5' TTTGTCGACATGAAYCCAAATCAAAARATAATAAC 3'
AB262 (32 mer)(SEQ ID N°7)
   5' TTTGGATCCYTACATCTTRTCGATGTCAAAGG 3'
pour isoler le gène codant pour la glycoprotéine neuraminidase (NA) du virus de la grippe équine (souche Newmarket 2/93) (Figure N°3, SEQ ID N°8) sous la forme d'un fragment PCR d'environ 1430 pb. Ce fragment a été purifié, puis digéré avec les enzymes de restriction SalI et BamHl pour isoler un fragment Sall-BamHl de 1418 pb contenant le gène EIV NA Newmarket 2/93. Ce fragment a ensuite été ligaturé avec le plasmide pVR1012 (voir exemple 7), préalablement digéré par SalI et BamHI, pour donner le plasmide pAB142 (6287 pb).

### Exemple 11 : Construction du plasmide pPB246 (gène EIV NA Kentucky 1/94)

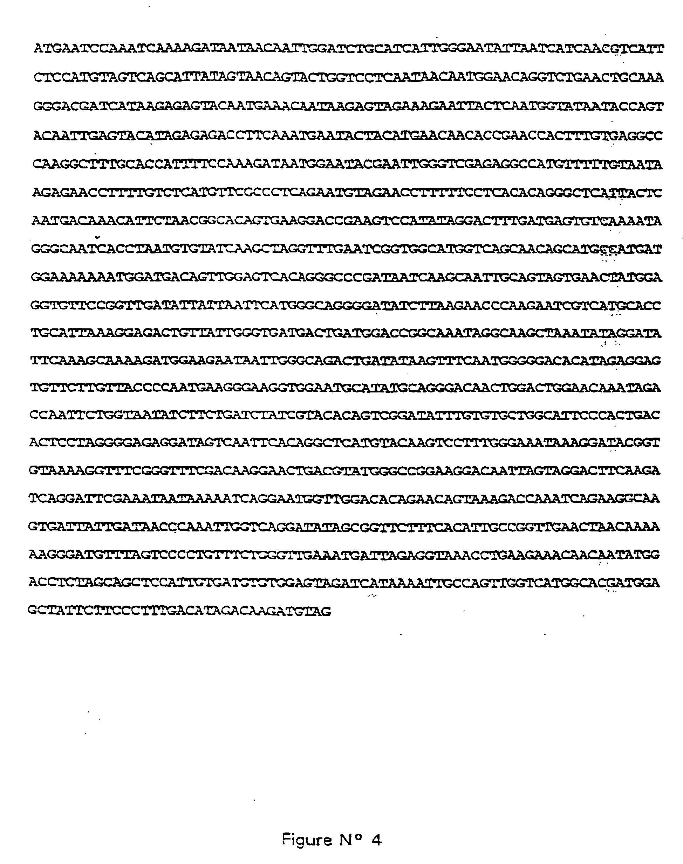

Une réaction de RT-PCR selon la technique décrite dans l'exemple 6 a été réalisée avec l'ARN génomique du virus de la grippe équine (Equine Influenza Virus ou EIV)(souche Kentucky 1/94)(Daly *et al.* J. Gen. Virol. 1996. **77.** 661-671, préparé selon la technique décrite dans l'exemple 4, et avec les oligonucléotides suivants AB260 et AB262 (exemple 10) pour isoler le gène codant pour la glycoprotéine neuraminidase (NA) du virus de la grippe équine (souche Kentucky 1/94) (Figure N°4, SEQ ID N°9) sous la forme d'un fragment PCR d'environ 1430 pb. Ce fragment a été purifié, puis digéré avec les enzymes de restriction SalI et BamHI pour isoler un fragment Sall-BamHl de 1418 pb contenant le gène EIV NA Kentucky 1/94. Ce fragment a ensuite été ligaturé avec le plasmide pVR1012 (voir exemple 7), préalablement digéré par SalI et BamHI, pour donner le plasmide pAB116 (6287 pb).

### Exemple 12 : Construction du plasmide pPB245 (gène EIV NP Newmarket 2/93)

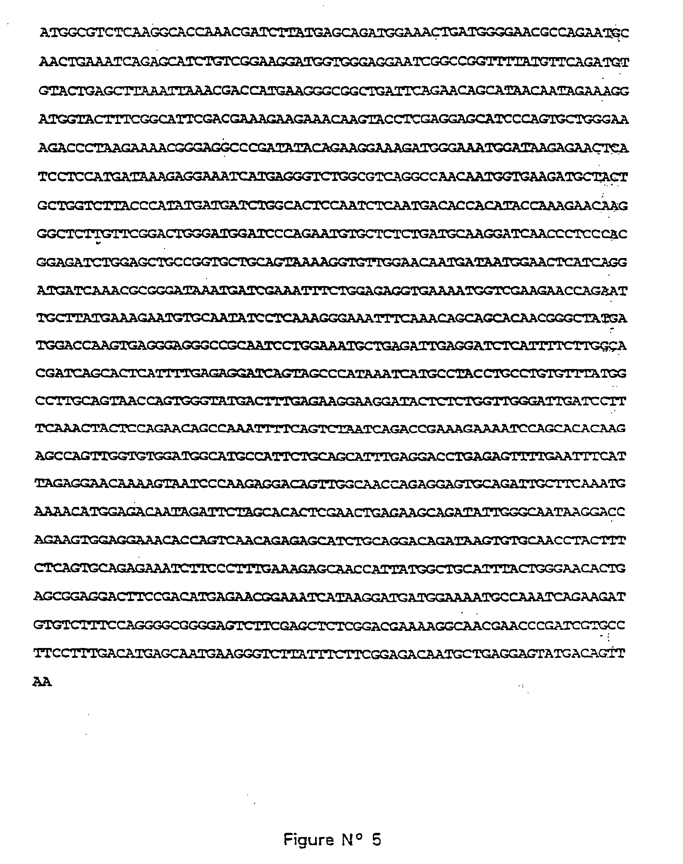

Une réaction de RT-PCR selon la technique décrite dans l'exemple 6 a été réalisée avec l'ARN génomique du virus de la grippe équine (Equine Influenza Virus ou EIV)(souche Newmarket 2/93)(Daly *et al.* J. Gen. Virol. 1996. **77**. 661-671, préparé selon la technique décrite dans l'exemple 4, et avec les oligonucléotides suivants :
CCL019 (25 mer)(SEQ ID N°10)
   5' TTGTCGACCATGGCGTCTCAAGGCAC 3'
CCL021 (28 mer)(SEQ ID N°11)
   5' TTTCTAGACTTTAAYTGTCAWACTCYTC 3'
pour isoler le gène codant pour la nucléoprotéine (NP) du virus de la grippe équine (souche Newmarket 2/93) (Figure N°5, SEQ ID N°12) sous la forme d'un fragment PCR d'environ 1520 pb. Ce fragment a été purifié, puis digéré avec les enzymes de restriction SalI et Xbal pour isoler un fragment Sall-Xbal de 1506 pb contenant le gène EIV NP Newmarket 2/93. Ce fragment a ensuite été ligaturé avec le plasmide pVR1012 (voir exemple 7), préalablement digéré par SalI et XbalI, pour donner le plasmide pPB245 (6389 pb).

### Exemple 13 : Construction du plasmide pPB246 (gène EIV NP Kentucky 1/94)

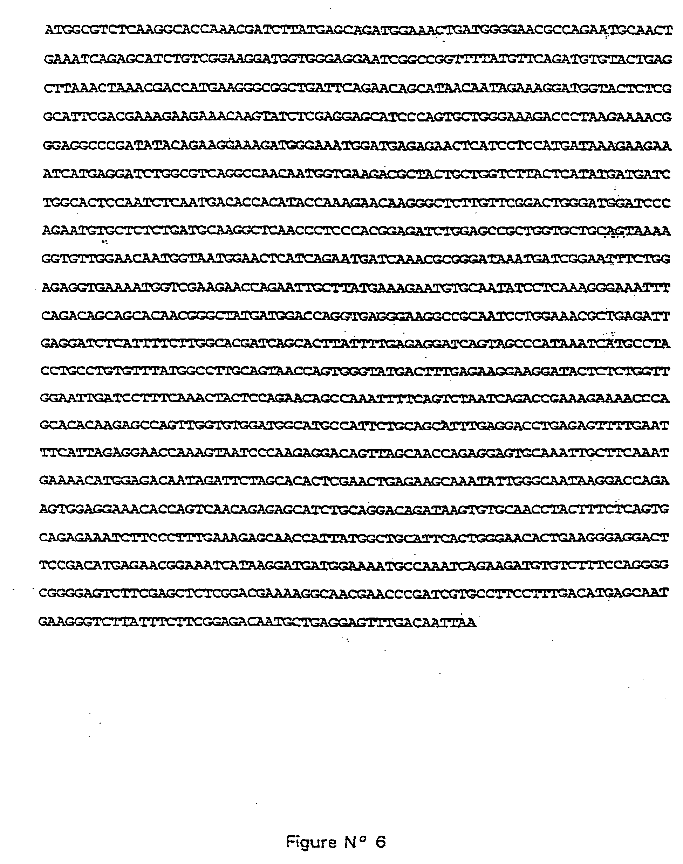

Une réaction de RT-PCR selon la technique décrite dans l'exemple 6 a été réalisée avec l'ARN génomique du virus de la grippe équine (Equine Influenza Virus ou EIV)(souche Kentucky 1/94)(Daly *et al.* J. Gen. Virol. 1996. **77**. 661-671, préparé selon la technique décrite dans l'exemple 4, et avec les oligonucléotides suivants CCL019 et CCL021 (exemple 12) pour isoler le gène codant pour la nucléoprotéine (NP) du virus de la grippe équine (souche Kentucky 1/94) (Figure N°6, SEQ ID N°13) sous la forme d'un fragment PCR d'environ 1520 pb. Ce fragment a été purifié, puis digéré avec les enzymes de restriction SalI et Xbal pour isoler un fragment Sall-Xbal de 1506 pb contenant le gène EIV NP Kentucky 1/94. Ce fragment a ensuite été ligaturé avec le plasmide pVR1012 (voir exemple 7), préalablement digéré par SalI et XbalI, pour donner le plasmide pPB246 (6389 pb).

### Exemple 14 : Construction du plasmide pPB156 (gène BHV-1 gB)

Sa construction est décrite dans WO-A-98 03200.

### Exemple 15 : Construction du plasmide pAB087 (gène BHV-1 gD)

Sa construction est décrite dans WO-A-98 03200.

### Exemple 16 : Construction du plasmide pAB090 (gène PRV gB)

Sa construction est décrite dans WO-A-98 03658.

### Exemple 17 : Construction du plasmide pPB098 (gène PRV gD)

Sa construction est décrite dans WO-A-98 03658.

### Exemple 18 : Construction du plasmide pAB044 (gène CDV HA)

Sa construction est décrite dans WO-A-98 03199.

### Exemple 19 : Construction du plasmide pAB036 (gène CDV F)

Sa construction est décrite dans WO-A-98 03199.

### Exemple 20 : Construction du plasmide pAB041 (gène G du virus de la rage)

Sa construction est décrite dans WO-A-98 03199.

### Exemple 21 : Application chez le cheval

Le vaccin testé est un mélange des 3 plasmides pCCL027 (exemple 8), pAB142 (exemple 10) et pPB245 (exemple 12) contenant et exprimant respectivement les gènes HA, NA et NP du virus EIV souche Newmarket 2/93. Ce mélange est associé ou non au carbomère comme selon la présente invention.
Le protocole de vaccination/épreuve a été le suivant :

| Groupe | Nombre de chevaux | Vaccin | Diluant | Dose |
|---|---|---|---|---|
| A | 5 | pCCL027+pAB142+p PB245 | solution saline | 3 x 400 µg |
| B | 5 | pCCL027+pAB142+p PB245 | Carbopol® 974P | 3 x 400 µg |
| C | 6 | Vaccin commercial | --- | 1 dose commerciale |
| D (témoins) | 5 | --- | --- | --- |

Des poneys (Welsh Mountain ponies) de 7 à 8 mois d'âge, ne présentant pas d'anticorps détectables contre les virus H3N8 et H7N7, mesurés par le test SRH (pour Single Radial Hemolysis ou hémolyse radiale simple), ont été utilisés pour cette étude. Les poneys ont été répartis au hasard dans les 4 groupes.
Les chevaux ont été vaccinés à J0 et J35 par voie intramusculaire. Le vaccin commercial utilisé pour le groupe C a été administré aux chevaux sous un volume de dose de 1 ml.
Les poneys des groupes A et B ont reçu chacun 2 doses de 5 ml à J0 et J35 par injection intramusculaire profonde dans le cou.
A J56, trois semaines après la deuxième vaccination, chaque poney a été infecté par une exposition à un aérosol provenant d'environ 1 ml de liquide allantoïdien contenant un total de 10^{7,3} ElD50 de virus influenza A-equi-2/Sussex/89, en utilisant un nébulisateur de modèle ULTRA 2000 (De Vilbiss, Somerset PA), comme décrit par Mumford *et al.* Equine Vet. J. 1990. **22**. 93-98.

Après épreuve, les poneys ont été suivis pour observer les signes cliniques (établissement d'un score clinique) et pour la température. Des écouvillonnages nasaux ont été réalisés quotidiennement depuis le jour 0 de l'épreuve jusqu'au 10ème jour après épreuve pour mesurer la quantité de virus excrété par chaque cheval éprouvé. Enfin, des prises de sang ont été effectuées tout au long du protocole, avant et après épreuve (jours J0, J7, J14, J35, J49, J56, J63 et J70) afin de mesurer la cinétique d'apparition et le taux des anticorps SRH et IHA (anticorps hémagglutinants) pour chaque groupe vacciné.

### Exemple 22 : Application chez le porc

L'efficacité d'un vaccin plasmidique, associé ou non au carbomère, a été étudiée chez le porc dans un modèle de vaccination/épreuve de la maladie d'Aujeszky. Le vaccin testé est un mélange des 2 plasmides pAB090 (exemple 16) et pPB098 (exemple 17), comprenant et exprimant respectivement les gènes gB et gD du virus PRV. Le mélange a été associé ou non au carbomère comme selon la présente invention. Le protocole de vaccination/épreuve utilisé a été le suivant :

| Groupe | Nombre de porcs | Vaccin | Diluant | Dose |
|---|---|---|---|---|
| A | 6 | pPB098+pAB090 | solution saline | 2 x 200 µg |
| B | 6 | pPB098+pAB090 | Carbopol® 974P | 2 x 200 µg |
| C | 6 | Geskypur | --- | 1 dose commerciale |
| D (témoins) | 6 | --- | --- | --- |

A J0, les porcs des groupes A et B ont été vaccinés avec le mélange des plasmides pPB098 et pAB090 (200 µg de chaque plasmide), associé ou non avec le carbomère, par voie intramusculaire, et sous un volume de 2 ml.
Les porcs du groupe C ont reçu une injection du vaccin commercial Geskypur (vaccin sous-unités, MERIAL, Lyon, France) par voie intramusculaire sous un volume de 2 ml. Les porcs du groupe D n'ont pas été vaccinés.
A J21, tous les porcs ont été éprouvés avec 2 ml (à raison d'1 ml par narine) d'une suspension virale de la souche d'épreuve Aujeszky souche NlA3 (dilution au 1/5 d'une solution stock titrant 10^{8,25} DICC50/ml).

Après épreuve, les porcs ont été suivis pour la mortalité et le critère de delta G7 (pesées individuelles à J0 et J7 de l'épreuve). Des écouvillonnages nasaux sont réalisés quotidiennement de J0 à J14 de l'épreuve pour mesurer la quantité de virus excrété après épreuve.
Enfin, des prises de sang ont été effectuées à J0, J7, J14, J21 et J28 du protocole pour mesurer la cinétique et le taux des anticorps séroneutralisant le virus de la maladie d'Aujeszky (PRV). Les anticorps ELISA anti-PRV d'isotypes IgG1 et lgG2 ont également été mesurés dans les sérums récoltés chez les porcs vaccinés et non vaccinés.

### Exemple 23 : Application chez le bovin

L'efficacité d'un vaccin plasmidique, associé ou non au carbomère, a été étudiée chez le bovin dans un modèle de vaccination/épreuve de la rhinotrachéite infectieuse bovine (IBR) ou BHV-1. Le vaccin testé est un mélange des 2 plasmides pPB156 (exemple 14) et pAB087 (exemple 15) comprenant et exprimant respectivement les gènes gB et gD du virus BHV-1. Le mélange a été associé ou non au carbomère comme selon la présente invention. Le protocole de vaccination/épreuve utilisé a été le suivant :

| Groupe | Nombre de veaux | Vaccin | Diluant | Dose |
|---|---|---|---|---|
| A | 6 | pAB087+pPB156 | solution saline | 2 x 300 µg |
| B | 6 | pAB087+pPB156 | Carbopol® 974P | 2 x 300 µg |
| C | 6 | Ibepur | --- | 1 dose commerciale |
| D (témoins) | 6 | --- | --- | --- |

A J0, les veaux des groupes A et B ont été vaccinés avec le mélange de plasmide pPAB087 et pPB156 (300 µg de chaque plasmide), associé ou non avec le carbomère, par voie intramusculaire, et sous un volume de 5 ml.
Les veaux du groupe C ont reçu une injection du vaccin commercial Ibepur (vaccin sous-unités, MERIAL, Lyon, France) par voie intramusculaire sous un volume de 2 ml. Les veaux du groupe D n'ont pas été vaccinés.
A J21, les groupes A, B et C ont reçu une deuxième injection de vaccin selon les mêmes modalités qu'à J0.
A J35. les veaux ont été éprouvés avec 2,5 ml (à raison d'1,25 ml par narine) d'une suspension virale de la souche d'épreuve BHV-1 souche B901 (dilution au 1/5 d'une solution stock titrant 10^{8.15} DICC50/ml).
Après épreuve, les veaux ont été suivis pour les signes cliniques (établissement d'un score clinique). Des écouvillonnages nasaux ont été réalisés quotidiennement de J0 à J14 de l'épreuve pour mesurer la quantité de virus excrété après épreuve.
Enfin, des prises de sang ont été effectuées à J0, J7, J14, J21, J35 et J49 du protocole pour mesurer la cinétique et le taux des anticorps séroneutralisant le virus de la rhinotrachéite infectieuse bovine (BHV-1). Les anticorps ELISA anti-BHV-1 d'isotypes IgG1 et IgG2 ont également été mesurés dans ces les sérums récoltés chez les veaux vaccinés et non vaccinés.

### Exemple 24 : Application chez le chien

L'efficacité d'un vaccin plasmidique, associé ou non au carbomère, a été étudiée chez le chien dans un modèle de vaccination/épreuve de la maladie de Carré (CDV). Le vaccin testé est un mélange des 2 plasmides pAB044 (exemple 18) et pAB036 (exemple 19) comprenant et exprimant respectivement les gènes HA et F du virus CDV. Le protocole de vaccination/épreuve utilisé a été le suivant :

| Groupe | Nombre de chiens | Vaccin | Diluant | Dose |
|---|---|---|---|---|
| A | 6 | pAB036+pAB041 | solution saline | 2x200 µg |
| B | 6 | pAB036+pAB041 | Carbopol® 974P | 2x200 µg |
| C | 6 | EURICAN | --- | 1 dose commerciale |
| D (témoins) | 6 | --- | --- | --- |

Les chiens des groupes A, B, C ont été vaccinés à J0 et J28 par voie intramusculaire. Les chiens des groupes A et B ont reçu pour chaque vaccination une injection de solution plasmidique de 400 µg au total (2 x 200 µg) sous un volume de 1 ml.

Les chiens du groupe C ont été vaccinés avec le vaccin EURICAN (CHPPI2) qui est un vaccin commercialisé par Mérial, Lyon, France. Une dose commerciale contient environ 10⁴ pfu de la souche vaccinale CDV Onderstepoort ainsi que les valences pour la vaccination contre l'hépatite de Rubarth, la parvovirose canine et le virus parainfluenza de type 2.

L'épreuve a été effectuée à J49, par administration intracérébrale d'une dilution au 1/10ème de la souche d'épreuve CDV "Synder-Hill" (lot préparé et fourni par l'USDA, USA).

Un suivi clinique a été effectué quotidiennement pendant 21 jours après l'épreuve pour noter les signes (état général, symptômes oculo-nasaux, symptômes digestifs, symptômes nerveux température) (notation selon les règles de la Pharmacopée Européenne). Les chiens éprouvés ont également été pesés une fois par semaine.

La protection a été appréciée sur les critères suivants :
- scores cliniques moyens de chaque groupe
- niveau de virémie CDV après épreuve (mesure de la charge virale dans les lymphocytes à J56, J61, J66, J70).
- numération/formule sur prises de sang effectuées à J48, J54, J56, J59, J63 et J70 (soit jours -1, 5, 7, 10, 14 et 21 après épreuve).
- évolution pondérale après épreuve.

Pour tous ces critères , les taux moyens de chaque groupe ont également été comparés entre eux et au taux moyen du groupe témoin.

Des prises de sang ont été réalisées aux jours J0, J14, J28, J56 et J70 pour le titrage des anticorps ELISA et séroneutralisant le virus de la maladie de Carré.

## Revendications

1. Vaccin pour la vaccination du cheval, comprenant un ADN nu incorporant au moins un acide nucléique codant pour un polypeptide antigénique d'un agent pathogène du cheval et exprimant *in vivo* ledit polypeptide antigénique, et au moins un composé adjuvant choisi parmi les polymères de l'acide acrylique ou méthacrylique et les copolymères d'anhydride maléique et d'éthylène.

2. Vaccin selon la revendication 1, comprenant un polymère de l'acide acrylique ou méthacrylique réticulé.

3. Vaccin selon la revendication 2, dans lequel le polymère est réticulé par un éther polyalcénylique de sucre ou de polyalcool.

4. Vaccin selon la revendication 3, dans lequel le polymère est réticulé par un allyl saccharose ou par de l'allylpentaérythritol.

5. Vaccin selon la revendication 1, comprenant un carbomère.

6. Vaccin selon la revendication 1, comprenant un copolymère d'anhydride maléique et d'éthylène linéaire ou réticulé.

7. Vaccin selon l'une quelconque des revendications 1 à 6, dans lequel le composé adjuvant est présent dans le vaccin à raison de 0,01% à 2% P/V.

8. Vaccin selon la revendication 7, dans lequel la concentration en composé adjuvant est de 0,06 à 1% en P/V.

9. Vaccin selon la revendication 8, dans lequel la concentration en composé adjuvant est de 0,1 à 0,6% en P/V.

10. Vaccin selon l'une quelconque des revendications 1 à 9, dans lequel l'ADN nu est un plasmide circulaire.

11. Vaccin selon l'une quelconque des revendications 1 à 10. dans lequel l'agent pathogène du cheval est :
- virus de la rhinopneumonie équine
- virus de la grippe équine
- Cl. Tetani
- virus de l'encéphalite de l'Est
- virus de l'encéphalite de l'Ouest
- virus de l'encéphalite vénézuélienne.

## Claims

1. A vaccine for vaccination of a horse, comprising a naked DNA incorporating at least one nucleic acid coding for an antigenic polypeptide of an agent pathogenic to a horse and expressing said antigenic polypeptide *in vivo*, and at least one adjuvant compound selected from polymers of acrylic or methacrylic acid and copolymers of maleic anhydride and ethylene.

2. A vaccine according to claim 1, comprising a cross-linked acrylic or methacrylic acid polymer.

3. A vaccine according to claim 2, in which the polymer is cross-linked by a polyalkenyl ether of sugar or polyalcohol.

4. A vaccine according to claim 3, in which the polymer is cross-linked by an allyl saccharose or by allylpentaerythritol.

5. A vaccine according to claim 1, comprising a carbomer.

6. A vaccine according to claim 1, comprising a linear or cross-linked copolymer of maleic anhydride and ethylene.

7. A vaccine according to any one of claims 1 to 6, in which the adjuvant compound is present in the vaccine in an amount of 0.01 % to 2% w/v.

8. A vaccine according to claim 7, in which the concentration of adjuvant compound is 0.06% to 1% w/v.

9. A vaccine according to claim 8, in which the concentration of adjuvant compound is 0.1% to 0.6% w/v.

10. A vaccine according to any one of claims 1 to 9, in which the naked dna is a circular plasmid.

11. A vaccine according to any one of claims 1 to 10, in which the agent which is pathogenic to a horse is:
• equine rhinopneumonia virus;
• equine influenza virus;
• Cl. Tetani;
• Eastern encephalitis virus;
• Western encephalitis virus;
• Venezuelan encephalitis virus.

## Patentansprüche

1. Pferdeimpfstoff, eine nackte DNA umfassend, die mindestens eine Nucleinsäure enthält, die ein antigenes Polypeptid eines Erregers einer Pferdekrankheit codiert und das antigene Polypeptid *in vivo* exprimiert, sowie mindestens ein Adjuvans umfassend, das ausgewählt ist aus der Gruppe der Acrylsäure- oder Methacrylsäurepolymere und der Maleinsäureanhydrid-Ethylen-Copolymere.

2. Impfstoff nach Anspruch 1, der ein vernetztes Polymer der Acrylsäure oder Methacrylsäure umfasst.

3. Impfstoff nach Anspruch 2, wobei das Polymer mittels eines Polyalkenylethers von Zucker oder Polyalkohol vernetzt ist.

4. Impfstoff nach Anspruch 3, wobei das Polymer mittels einer Allylsaccharose oder mit Hilfe von Allylpentaerythrit vernetzt ist.

5. Impfstoff nach Anspruch 1, der ein Carbomer umfasst.

6. Impfstoff nach Anspruch 1, der ein lineares oder vernetztes Maleinsäureanhydrid-Ethylen-Copolymer umfasst.

7. Impfstoff nach einem der Ansprüche 1 bis 6, wobei das Adjuvans in einem Verhältnis von 0,01% bis 2% G/V im Impfstoff vorhanden ist.

8. Impfstoff nach Anspruch 7, wobei die Konzentration des Adjuvans 0,06 bis 1% G/V beträgt.

9. Impfstoff nach Anspruch 8, wobei die Konzentration des Adjuvans 0,1 bis 0,6% G/V beträgt.

10. Impfstoff nach einem der Ansprüche 1 bis 9, wobei die nackte DNA ein zirkuläres Plasmid ist.

11. Impfstoff nach einem der Ansprüche 1 bis 10, wobei der Erreger der Pferdekrankheit eines der folgenden Viren ist:
- Virus der equinen Rhinopneumonitis
- Virus der Pferdegrippe
- Clostridium tetani
- Virus der Eastern-Equine-Encephalitis
- Virus der Western-Equine-Encephalitis
- Virus der Venezuelan-Equine-Encephalitis
